# EUROPÄISCHE PATENTSCHRIFT

(11) **EP 1 119 319 B1**
(45) Veröffentlichungstag und Bekanntmachung des Hinweises auf die Patenterteilung: **18.12.2002**
(21) Anmeldenummer: 00936758.2
(22) Anmeldetag: 20.05.2000
(51) Int. Cl.: A61F 2/28, A61F 2/80

(54) **OBERSCHENKELSTUMPFENDOPROTHESE FÜR EINE EXOPROTHETISCHE VERSORGUNG**
THIGH STUMP ENDOPROSTHESIS FOR AN EXOPROSTHETIC TREATMENT
ENDOPROTHESE DE MOIGNON DE CUISSE POUR EXOPROTHESE

(30) Priorität: 14.07.1999 DE 19932388
(43) Veröffentlichungstag der Anmeldung: 01.08.2001
(73) Patentinhaber: ESKA Implants GmbH & Co., 23556 Lübeck (DE)
(72) Erfinder: GRUNDEI, Hans, D-23558 Lübeck (DE)
(74) Vertreter: Weiss, Christian, Dipl.-Ing.
(86) Internationale Anmeldenummer: EP0004552
(87) Internationale Veröffentlichungsnummer: WO01005335

(56) Entgegenhaltungen:
- EP-A- 0 577 178
- EP-A- 0 639 351
- WO-A-91/07932
- DE-A- 3 125 268
- DE-A- 4 338 746
- DE-A- 19 630 298
- DE-A- 19 826 638
- DE-C- 19 857 907
- GB-A- 2 231 271
- US-A- 3 947 897
- US-A- 4 158 895
- US-A- 5 549 682
- US-A- 5 766 251

## Beschreibung

Die vorliegende Erfindung betrifft eine Oberschenkelstumpf-Endoprothese für eine exoprothetische Versorgung eines im Oberschenkelbereich beinamputierten Patienten. Dabei ist der Oberschenkelstumpf in einen Schaft ziehbar, dem sich die Nachbildung eines Knie-, Unter- und Fußteils der Prothese anschließt. Die Endoprothese weist einen proximalen Stielteil auf, das in einen Femurstumpf setzbar ist.

Eine solche Endoprothese ist aus der Druckschrift DE-A-31 25 268 bekannt.

Der vorliegenden Erfindung liegt die Aufgabe zugrunde, das Wahrnehmungsgefühl des Patienten noch weiterhin zu steigern.

Diese Aufgabe wird mit dem Gegenstand des Anspruchs 1 gelöst.

Erfindungsgemäß ist mit dem Kondylenersatz eine keilförmige Unterlage verbunden, welche die natürliche Valgität, d. h. also die Winkelstellung des Femurstumpfes in Richtung medial, wiederherstellt. Hierdurch werden die Kräfteverteilungen den entsprechenden Verteilungen in einem gesunden Bein nachempfunden. Dies bedeutet, daß mit dem Keil eine Parallelität zwischen der Unterseite des Keiles und den Gleitflächen eines künstlichen Kniegelenkes hergestellt werden kann unter weitgehender Beibehaltung des natürlichen CCD-Winkels. Eine Vergleichmäßigung der Belastung entlang der Prothesenachse wird hierdurch erzielt.

Aus dem Bereich der Chirurgie ist zwar bereits ein Implantat bekannt (US-A-5,766,251), welches als keilförmiges Ausgleichsstück ausgebildet ist, welches Korrekturen beispielsweise hinsichtlich des Valgitätswinkels gestattet. Der Einsatz des keilförmigen Implantates findet unter anderem statt im Gelenkbereich der Tibia, womit das Tibiaplateau dem Neigungswinkel des keilförmigen Implantates entsprechend einseitig angehoben wird. Einen Hinweis auf die Verwendung dieses Implantates bei der Versorgung eines beinamputierten Patienten ist dieser Druckschrift allerdings nicht zu entnehmen.

Gemäß einer bevorzugten Ausführungsform liegt der Keilwinkel in einem Bereich zwischen 5 und 9°. Die 5° werden vorzugsweise bei männlichen Patienten, die 9° maximal bei weiblichen Patienten zur Anwendung kommen.

Bevorzugt ist die Unterlage mit der stoßdämpfenden Schicht der Oberschenkelstumpf-Endoprothese verbunden.

Besonders bevorzugt wird die Ausbildung der Unterlage aus einem stoßdämpfenden Kissen, so daß Belastungen nicht direkt in den Femurstumpf eingeleitet werden. Zu diesem Zwecke ist die Unterlage bevorzugt aus Silikon hergestellt.

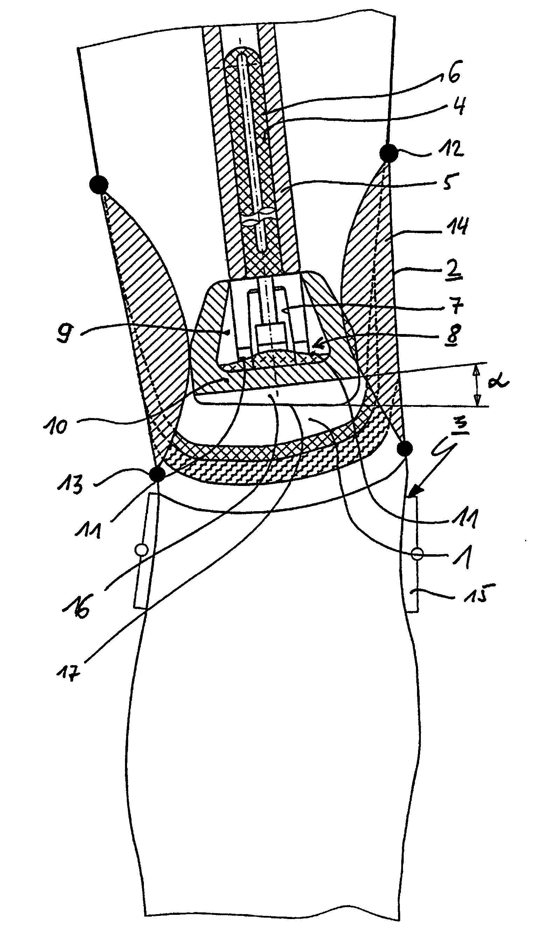

Die Erfindung wird anhand eines Ausführungsbeispiels gemäß der einzigen Zeichnungsfigur beispielhart näher erläutert.

Die einzige Zeichnungsfigur zeigt schematisch einen Oberschenkelstumpf 1 mit einer in dem Femurstumpf 5 implantierten Oberschenkelstumpf-Endoprothese.

Ein intramedullärer Stiel 4 bildet das proximale Stielteil, welches in den Markraum des Femurstumpfes gesetzt ist. Die Oberfläche des Stielteils 4 ist mit einer offenmaschigen, dreidimensionalen Raumnetzstruktur 6 belegt, durch welche hindurch Knochentrabekel wachsen können, so daß das Stielteil 4 nach einer gewissen Einheilphase, was den Substratfluß betrifft, quasi Bestandteil des natürlichen Knochens geworden ist.

Dem Stielteil 4 schließt sich distalseitig ein konischer Adapter 7 an. Mittels dieses konischen Adapters 7 ist distalseitig der Kondylenersatz 8 mit dem Stielteil 4 gekoppelt.

Das Kondylenersatzteil 8 ist so ausgebildet, daß es die Form 11 natürlicher Kondylen eines Kniegelenks nachempfindet.

Der Kondylenersatz 8 ist vorliegend mit einer stoßdämpfenden Schicht 10 aus Silikon beschichtet.

Mit dem Kondylenersatz 8 bzw. mit der deckenden stoßdämpfenden Schicht verbunden ist die Unterlage 16. Die Unterlage 16 ist keilförmig ausgebildet und weist einen Keilwinkel zur Horizontalen im Bereich von 5° < α < 9° auf.

Die Unterlage 16 besteht vorliegend aus einem dämpfenden Kissen und unterstützt somit im gezeigten Ausführungsbeispiel die Abdämpfung von Stoßbelastungen auf die Knie-, Unterschenkel- und Fußteile 3. Der Keilwinkel α der Unterlage 16 wird patientenindividuell bestimmt, so, daß die Unterseite 17 der Unterlage 16 im wesentlichen parallel verläuft zu der Ebene der Gleitbahnen der künstlichen Kondylen eines angekoppelten künstlichen Kniegelenks.

Hierdurch wird ein hohes Maß an einer Gleichverteilung der Belastungen in der Gesamtprothese erreicht.

## Patentansprüche

1. Oberschenkelstumpf-Endoprothese für eine exoprothetische Versorgung eines im Oberschenkelbereich beinamputierten Patienten, wobei der Oberschenkelstumpf (1) in einen Schaft (2) ziehbar ist, dem sich die Nachbildung eines Knie-, Unterschenkel- und Fußteils (3) der Prothese anschließt, aufweisend ein proximales Stielteil (4), das in einen Femurstumpf (5) setzbar ist, **dadurch gekennzeichnet, dass** das Stielteil (4) zumindest teilweise mit einer offenmaschigen, dreidimensionalen Raumnetzstruktur (6) bedeckt ist und an seinem distalen Ende einen konischen Adapter (7) aufweist, mittels dessen ein Kondylenersatz (8), der der Form (11) natürlicher Kondylen eines Kniegelenks nachgebildet ist, an das Stielteil (4) gekoppelt ist, und dass die Endoprothese eine keilförmige Unterlage (16) aufweist, die mit dem Kondylenersatz (8) verbunden ist.

2. Oberschenkelstumpf-Endoprothese nach Anspruch 1, bei der der Keilwinkel α der Unterlage (16) im Bereich 5°<α<9° liegt.

3. Oberschenkelstumpf-Endoprothese nach Anspruch 1 oder 2, bei der die Unterlage (16) mit der stoßdämpfenden Schicht (10) verbunden ist.

4. Oberschenkelstumpf-Endoprothese nach einem der Ansprüche 1 bis 3, bei der die Unterlage (16) aus einem stoßdämpfenden Kissen besteht.

5. Oberschenkelstumpf-Endoprothese nach Anspruch 4, bei der die Unterlage aus Silikon besteht.

## Claims

1. Thigh stump endoprosthesis for exoprosthetic care of a patient with leg amputation in the thigh region, wherein the thigh stump (1) can be drawn into a shaft (2), to which the imitation of a knee, lower limb and foot part (3) of the prosthesis is connected, having a proximal stem part (4), which can be placed in a femoral stump (5), **characterised in that** the stem part (4) is covered at least partly by an open-meshed, three-dimensional spatial network structure (6) and has at its distal end a conical adapter (7), by means of which a condyle replacement (8) which imitates the shape (11) of natural condyles of a knee joint, is coupled to the stem part (4), and **in that** the endoprosthesis has a wedge-shaped base (16) which is connected to the condyle replacement (8).

2. Thigh stump endoprosthesis according to claim 1, in which the wedge angle α of the base (16) lies in the range 5° < α < 9°.

3. Thigh stump endoprosthesis according to claim 1 or 2, in which the base (16) is connected to the shock-absorbing layer (10).

4. Thigh stump endoprosthesis according to one of claims 1 to 3, in which the base (16) consists of a shock-absorbing cushion.

5. Thigh stump endoprosthesis according to claim 4, in which the base consists of silicone.

## Revendications

1. Endoprothèse de moignon de cuisse pour appareillage exoprothétique d'un patient amputé de la jambe au niveau de la cuisse, moyennant quoi le moignon de cuisse (1) peut se rétracter dans un fût, lequel est raccordé au substitut de genou, de jambe ou de pied (3) de la prothèse, présentant une section de tige proximale (4), laquelle peut être implantée dans un moignon fémoral (5), **caractérisée par le fait que** la section de tige (4) est recouverte, du moins en partie, par une structure en filet tridimensionnelle avec de larges mailles (6) et présentant à son extrémité distale un adaptateur conique (7), au moyen duquel un substitut condylien (8), lequel est constitué de telle façon qu'il reproduise la forme de condyles naturels d'une articulation du genou, est couplé à la section de tige (4), et **par le fait que** l'endoprothèse présente un support cunéiforme (16) qui est raccordé au substitut condylien (8).

2. Endoprothèse de moignon de cuisse selon la revendication 1, dans laquelle l'angle cunéiforme α du support (16) se situe dans la plage de 5 <α<9°.

3. Endoprothèse de moignon de cuisse selon la revendication 1 ou 2, dans laquelle le support (16) est relié à la couche amortissante (10).

4. Endoprothèse de moignon de cuisse selon l'une des revendications 1 à 3, dans laquelle le support (16) est constitué d'un coussin amortisseur.

5. Endoprothèse de moignon de cuisse selon la revendication 4, dans laquelle le support est fait en silicone.
